# EUROPEAN PATENT APPLICATION

(11) **EP 3 103 435 A1**
(43) Date of publication of application: **14.12.2016**
(21) Application number: 15746299.5
(22) Date of filing: 27.01.2015
(51) Int. Cl.: A61K 8/41, A61K 8/33, A61K 8/30, A61Q 7/00, A61Q 5/00

(54) **COMPOSITION FOR PROMOTING HAIR GROWTH OR PREVENTING HAIR LOSS**

(30) Priority: 04.02.2014 KR 20140012479; 19.01.2015 KR 20150008523
(71) Applicant: Neopharm Co., Ltd., Daejeon 305-510 (KR)
(72) Inventor: JEONG, Se Kyoo, Daejeon 302-734 (KR); BAE, Jong Hwan, Daejeon 305-751 (KR); PARK, Bu Mahn, Daejeon 302-749 (KR)
(74) Representative: dompatent von Kreisler Selting Werner - Partnerschaft von Patent- und Rechtsanwälten mbB
(86) International application number: PCT/KR2015/000820
(87) International publication number: WO 2015/119393

(57) **Abstract**

The present invention relates to a composition for promoting hair growth, preventing hair loss, or relieving inflammation, and more specifically, to a composition which has excellent stability to the skin, has no side effects, and exhibits excellent effects of hair growth promotion and hair loss prevention by promoting hair growth and delaying hair loss. The composition according to the present invention contains a material belonging to a ceramidase inhibitor, glycol, C1-C5 lower alcohol, and water, and thus the composition is safe, has no side effects, can be continuously used for a long period of time, has an excellent effect of hair growth promotion or hair loss prevention by significantly promoting hair growth, and exhibits an inflammation relieving effect as well as a hair growth promoting effect, additionally.

## Description

### [Technical Field]

The present invention relates to a composition for promoting hair growth, preventing hair loss, or relieving inflammation, and more specifically, to a composition which has excellent stability to a skin, has no side effects, and exhibits excellent effects of hair growth promotion and hair loss prevention by promoting hair growth and delaying hair loss.

### [Background Art]

A main cause of hair loss is a genetic factor. However, recently, people suffering from hair loss have been gradually increased by environmental pollution, westernized dietary habits such as processed foods, frequent perming and dyeing, bad scalp care, etc., in addition to an increase in social stress.

Hair is maintained by repeating hair growth and hair loss in a cycle of anagen, catagen and telogen. Specifically, the cycle may be divided into the anagen in which hair grows, the catagen in which growth is completed and a hair bulb part is reduced, the telogen in which papilla stops an activity and hair is maintained on the scalp, and a development stage in which the papilla starts the activity or generates new hair to lose old hair.

A period of the anagen of hair is about three to five years for a man, and four to six years for a woman, and a period of the catagen is about 30 to 45 days, and a period of the telogen is about three to four months, and accordingly, hair loss is naturally caused. In addition, at the end of the telogen, the development stage that new hair is generated starts.

The hair loss is a normal phenomenon. However normal people have a large number of hairs in an anagen state, and on the contrary, people with alopecia havae a large number of hairs in the telogen state, such that a hair loss phenomenon is visually noticeable.

People with alopecia are characterized by miniaturization of hair. As hair loss proceeds, a period of the anagen is reduced, and accordingly, the miniaturization of hair also proceeds. Therefore, in order to treat hair loss, it is important to induce hair follicles from the telogen into the anagen quickly, and to increase shorten anagen.

Male pattern alopecia is a phenomenon caused by the male hormone called testosterone which is a hormone that shows male sexual characteristics and helps with the development of muscle, development of male organs, etc., in adolescence When this testosterone is changed into stronger dihydrotestosterone (DHT) by 5 α-reductase, this hormone acts on the hair follicles by inducing the hair follicles from the anagen into the catagen, such that hair loss is caused. Therefore, in order to treat alopecia caused by the reason, a method of inhibiting production of DHT by 5 α-reductase is mainly used.

Female pattern alopecia is mainly caused by reduction of an amount of estrogen after menopause. In the female pattern alopecia, hair loss does not occur in a front part of a head, but mainly occurs in a middle part of a head, which is unlike the male pattern alopecia. An effect of 5 α-reductase on the female pattern alopecia is smaller than that on the male pattern alopecia. Therefore, drugs inhibiting 5 α-reductase are not significantly effective for women with alopecia after menopause. Accordingly, minoxidil or estrogen is primarily used as therapeutic agents for alopecia.

Alopecia areata is caused by autoimmune diseases, mental stress, or genetic predisposition. The alopecia areata includes circular or ovoid hair loss and ringworm or trichotillomania. A cause of the alopecia areata is fundamentally different from that of androgenetic alopecia, and a treatment method thereof is also different from that of androgenetic alopecia, and accordingly, a method of treating adrenocortical hormone is used, or a method of applying minoxidil to an affected area or artificially causing stimulation to the affected area is used.

With regard to various and complex causes of hair loss as described above, currently known hair loss prevention products including components that aim at promoting blood circulation, inhibiting male hormone action, enhancing hair root function, etc., as active components, are being sold on the market. However, among them, products having appreciable effect do not exist yet, and side effects are raised in most of products.

As hair growth products, Minoxidil (6-amino-1,2-dihydro-1-hydroxy-2-imino-4-phenoxypyrimidine) (U.S. Patent No. 3,382,247) was initially developed for the purpose of promoting blood circulation, and then proved a hair growth effect as a side effect among patients using Minoxidil, and after that, received approval from the U.S. Food and Drug Administration (FDA) as raw material for hair growth. Even now, Minoxidil has been used as a therapeutic agent for hair growth. Finasteride (Merck, U.S. Patent No. 5,215,894) was initially used as a prostate treatment for men, but has also been used as a therapeutic agent for male pattern alopecia by controlling a dose in a human body.

However, it has been reported that Minoxidil has side effects such as stickiness and skin irritation, and Finasteride which is orally administered at present has side effects such as sexual dysfunction, etc., depending on its intake, and is effective for hair loss only when being steadily taken. In addition, the effect may not be expected when applying 5 α-reductase activation inhibitors thereof, but be expected only when orally administrating 5 α-reductase activation inhibitors thereof, such that there are a lot of inconvenience in use.

### [Disclosure]

### [Technical Problem]

An object of the present invention is to provide a composition capable of remarkably promoting hair growth, preventing hair loss, and preventing dry skin (xeroderma) and symptoms of skin aging without causing problems, the composition including: a material belonging to a ceramidase inhibitor; glycol; C1-C5 lower alcohol; and water.

### [Technical Solution]

In one general aspect, a composition for promoting hair growth, preventing hair loss, or relieving inflammation, includes a ceramidase inhibitor; 500 to 2000 parts by weight of glycol; 400 to 1500 parts by weight of C1-C5 lower alcohol; and 100 to 1000 parts by weight of water, based on 100 parts by weight of the ceramidase inhibitor.

### [Advantageous Effects]

The composition according to the present invention contains a material belonging to a ceramidase inhibitor, glycol, C1-C5 lower alcohol, and water, such that the composition is safe, has no side effects, is continuously usable for a long period of time, and has an excellent effect of hair growth promotion or hair loss prevention by significantly promoting hair growth. Further, the composition exhibits an effect of relieving inflammation as well as an effect of promoting hair growth.

### [Description of Drawings]

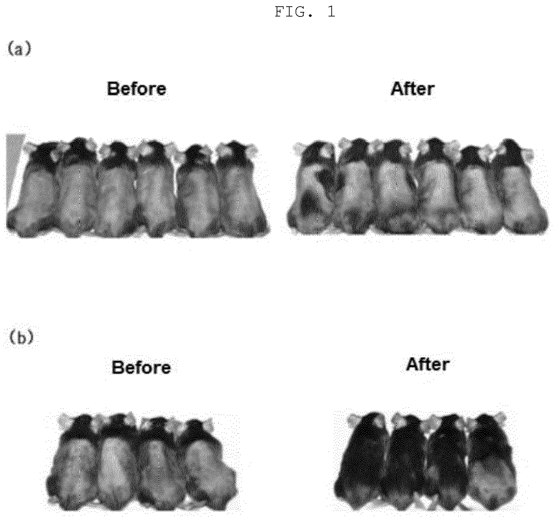

FIG. 1 illustrates comparison between a composition for promoting hair growth of the present invention and Control Group in view of an effect of promoting hair growth. Specifically, FIG. 1(a) illustrates a case where only ethanol is applied according to Comparative Example 1, and FIG. 1(b) illustrates a case where the composition for promoting hair growth of Example 1 is applied.

### [Best Mode]

The present invention relates to a composition for promoting hair growth, preventing hair loss, or relieving inflammation, including: a ceramidase inhibitor; 500 to 2000 parts by weight of glycol; 400 to 1500 parts by weight of C1-C5 lower alcohol; and 100 to 1000 parts by weight of water, based on 100 parts by weight of the ceramidase inhibitor.

It is known that the ceramidase inhibitor prevents dry skin, maintains and/or promotes moisture retention, prevents skin aging, maintains and/or restores skin barrier function, and promotes formation of mature corneocytes, strengthen epithelial differentiation, and maintains and/or increases epidermal lipid levels.

In the present invention, the ceramidase inhibitor is not limited, but may include N-oleylethanolamine, derivatives thereof, salts thereof, or mixtures thereof.

N-oleylethanolamine, derivatives thereof, or salts thereof are more preferred since hair growth may be very remarkably promoted. In addition, when N-oleylethanolamine, derivatives of N-oleylethanolamine, salts of N-oleylethanolamine, or mixtures thereof among the ceramidase inhibitors are included, it is more preferred to obtain an effect of inhibiting inflammation. Here, the derivatives of N-oleylethanolamine may have a salt form.

The ratio of the composition is a range in which skin irritation is inhibited when being applied even without inhibiting an effect of hair growth, a refreshing feeling by appropriate volatilization is provided while securing a use time required for uniform application, and absorption of the material is capable of being promoted. In particular, when a composition ratio of the ceramidase inhibitor and the glycol is out of the above-described range, it may be confirmed from an exemplary embodiment of the present invention that an effect of hair growth is remarkably deteriorated.

The composition in the present invention is not limited, but may contain 0.1 to 10 wt% of the ceramidase inhibitor, preferably, 1 to 10 wt%, more preferably, 1 to 8 wt%, and more preferably, 4 to 6 wt% of the ceramidase inhibitor to have a remarkable hair growth effect by a small number of applications such as once to three times per day.

In the present invention, the glycol is at least one material selected from polyethylene glycol, polypropylene glycol and polyethylene propylene glycol, and preferably, may be polypropylene glycol. The glycol may be prepared into paste, cream or gel formulations, and may improve moisture. Here, it is preferred that a weight average molecular weight (Mw) of the glycol is a low molecular weight of 50 to 150.

The C1-C5 lower alcohol may be a solvent of the ceramidase inhibitor, and may be at least one material selected from methanol, ethanol, acetone, isopropyl alcohol, and n-butanol. The lower alcohol may have excellent volatility to promote the feeling when the composition is applied, and in particular, ethanol is more preferable since it has excellent volatility and is harmless. Water may supply moisture to skin and promote skin absorption when the composition is applied.

The composition in the present invention may be a pharmaceutical composition, a cosmetic composition or a sanitary aid composition.

The formulation of the composition in the present invention is not limited, but may be a skin external preparation including solution, cream, ointment, paste, aerosol agent, gel or wax, or a hair external preparation including shampoo, rinse, ampoule or treatment.

In the formulation, the composition according to an exemplary embodiment of the present invention may be the skin external preparation or the hair external preparation, wherein the skin external preparation may include a form of solution, cream, ointment, paste, aerosol agent, gel or wax, and the hair external preparation may include a form of shampoo, rinse, ampoule or treatment.

In the skin external preparation, the skin may include all epidermis having hair root and hair follicles throughout the whole body of scalp, chins, armpits, the root of the ear, cheeks, eyebrows, etc. In the case of the hair external preparation, hair may include hair in the whole body of hair, eyebrows (including eyelashes and eyebrows), beard, armpits, etc. Here, the external preparation may mean that the composition is directly applied to or distributed on the epidermis and/or hair. Here, the epidermis and/or hair may include epidermis and/or hair of mammal including human.

When the composition according to an exemplary embodiment of the present invention is a pharmaceutical composition, the derivatives of N-oleylethanolamine may be a pharmaceutically acceptable salt, and the pharmaceutically acceptable salt may mean a salt prepared by a conventional method in the art, and a preparation method thereof is known to a person in the art. Specifically, the pharmaceutically acceptable salt may include salts derived from inorganic acids and organic acids and bases that are pharmacologically acceptable or physiologically acceptable, but examples of the pharmaceutically acceptable salt are not limited thereto.

When the composition according to an exemplary embodiment of the present invention is a pharmaceutical composition, the composition may be a pharmaceutical composition for preventing hair loss.

When the composition according to an exemplary embodiment of the present invention is a pharmaceutical composition, the composition may include a pharmaceutically acceptable carrier and/or excipient. The pharmaceutically acceptable carrier may be used without limitation as long as it is generally used for pharmaceutical preparation. The carrier and/or excipient may be at least one selected from the group consisting of a binder, a lubricant, a disintegrant, a lubricant, a coating material, an emulsifier, a suspension, a solvent, a stabilizer, an absorption aid, a water for injection, and an isotonic agent. In addition, the composition may further include a filler, an anti-coagulant, a lubricant, a wetting agent, a flavoring agent, an emulsifier, a preservative, etc. Here, the formulation may be a form of granule, powder, solution, cream, ointment, aerosol, paste, gel, wax, etc., and the solution may include a suspension state or an emulsion state as well as a state in which the solution is dissolved in a solvent.

When the composition according to an exemplary embodiment of the present invention is a pharmaceutical composition, an appropriate amount at which the composition is applied may vary depending on state and body weight, degree of disease of target to be applied, formulation, an application route, and duration of the composition. In the composition according to an exemplary embodiment of the present invention, the composition containing the ceramidase inhibitor, preferably, N-oleylethanolamine, derivatives of N-oleylethanolamine, salts of N-oleylethanolamine or mixtures thereof, glycol, C1-C5 lower alcohol; and water, may be applied so that an amount per unit area of epidermis region to be applied is 10 to 30µl/cm² when the composition is applied once. The application may be performed once to six times per day, and when the application is performed twice or more per day, each application may be performed at the same time interval.

When the composition of the present invention is a cosmetic composition or a sanitary aid composition, the composition may contain adjuvants generally used for the cosmetic composition or the sanitary aid composition, for example, hydrophilic or lipophilic gelling agents, hydrophilic or lipophilic active agents, preservatives, antioxidants, solvents, fragrances, fillers, blocking agents, pigments, odor absorbing agents, dyes or mixtures thereof, in addition to a ceramidase inhibitor, preferably, N-oleylethanolamine, derivatives of N-oleylethanolamine, salts of N-oleylethanolamine, or mixtures thereof, glycol, C1-C5 lower alcohol, and water.

When the composition of the present invention is a cosmetic composition or a sanitary aid composition, the composition may be used into a formulation of cosmetics or sanitary aid. The sanitary aid formulation may be a form of granule, powder, solution, cream, ointment, aerosol, paste, gel, wax, etc., and the solution may include a suspension state or an emulsion state as well as a state in which the solution is dissolved in a solvent. The formulation of the cosmetics or the sanitary aid may be various such as solution, sol gel, emulsion, oil, wax, aerosol, etc., for example, hair tonic, hair cream, hair lotion, hair shampoo, hair rinse, hair conditioner, hair spray, hair aerosol, pomade, powder, gel, hair pack, hair treatment, eyebrow restorer, eyelash restorer, eyelash nutrient, pet shampoo, pet rinse, etc., but examples of the formulation are not limited thereto.

In addition, the composition according to an exemplary embodiment of the present invention may contain various components combined in general compositions for preventing hair loss or promoting hair growth so as to be appropriate for the above-described various formulations or final purposes. As an example, when the composition has a shampoo formulation, the composition may contain a synthetic surfactant which is a cleaning component and at least any one selected from preservatives, thickeners, viscosity modifiers, pH adjusters, fragrances, dyes, hair conditioning agents and water. The general various components combined in the general compositions for preventing hair loss or promoting hair growth so as to be appropriate for the above-described various formulations or final purposes are widely known to experts in the art.

Hereinafter, the present invention will be described in detail with reference to Examples. However, the Examples are provided to help a specific understanding of the present invention, and the protection scope of the present invention is not limited to the following Examples.

### [Preparation of N-oleylethanolamine]

100 mL of dichloromethane (DCM) was added to 5.0 g of ethanolamine (16.62 mmol), and 2.3 mL of triethylamine (16.62 mmol) was added thereto, thereby preparing a reaction solution. A temperature of the reaction solution was decreased to be 0°C by using an ice bath, and 13.8 mL of oleoyl chloride (CH₃(CH₂)₇CH=CH(CH₂)₇COCl, 0.1 mol) was slowly added to the reaction solution, followed by stirring at room temperature for 4 hours. After the stirring was completed, 150 mL of water was added, and the resulting solution was extracted with 150 mL of ethyl acetate, and an organic layer was washed with 150 mL of brine. All organic layers were collected and dried over sodium sulfate, and filtered filtrate was subjected to rotary evaporation to remove a solvent. The residue was washed with hexane to obtain 5.27 g of a white solid having a yield of 97%. MS (oleylethanolamine C₂₀H₃₉NO₂: 325.53) and NMR results of the obtained solid were as follows.

MS (ESI pos. ion) m/z: 326 (MH+). ¹H NMR (600 MHz, CDCl₃) : 5.92 (br s, 1H), 5.36 - 5.32 (m, 2H), 3.73 (t, J = 5.4 Hz, 2H), 3.42 (q, J = 5.4 Hz, 2H), 2.20 (t, J = 7.8 Hz, 2H), 2.02 - 1.99 (m, 4H), 1 . 66 - 1.61 (m, 2H), 1.31 - 1.27 (m, 21H), 0.88 (t, J = 7.2 Hz, 3H).

### [Example 1]

A composition for promoting hair growth was prepared by mixing 5 wt% of N-oleylethanolamine as prepared above, 30 wt% of ethanol, 50 wt% of polypropylene glycol (Mw=76.09), and 15 wt% of water.

An evaluation through *in vivo* experiment that was mainly used for hair growth and hair fostering was performed on a six-week old C57BL/6 mouse. The C57BL/6 mouse is an animal model which is the most widely used for testing hair growth and has a characteristic period in which hair enters into catagen from 6 weeks after birth, and into telogen from 7 weeks after birth, and goes back to anagen at 12 weeks after birth.

The mouse had an adaptation period of about 1 week under breeding conditions of 23 °C, relative humidity of 55 %, and illumination time for 12 hours, and then, was tested when the mouse was 7 weeks old.

The mouse was anesthetized for 1 minute in a desiccator containing ether, and then, hair on the back part thereof was removed by using a depilator. After 1 day, the prepared composition for promoting hair growth was applied on the mouse with an unscathed back once a day. For one application, the composition was applied in an amount of 20 µL per unit area (1 cm²) of skin. Then, change in color of the applied part was observed by taking images at an interval of one week.

### [Comparative Example 1]

Comparative Example 1 was performed in the same manner as Example 1 except for only using the same amount of ethanol (vehicle) instead of using the composition for promoting hair growth of Example 1.

### Comparative Example 2]

Comparative Example 2 was performed in the same manner as Example 1 except that a composition for promoting hair growth was prepared by mixing 5 wt% of N-oleylethanolamine and 95 wt% of ethanol.

### [[Comparative Example 3]

Comparative Example 3 was performed in the same manner as Example 1 except that a composition for promoting hair growth was prepared by mixing 4 wt% of N-oleylethanolamine, 43 wt% of ethanol, 18 wt% of polypropylene glycol (Mw=76.09), and 35 wt% of water.

### [Comparative Example 4]

Comparative Example 4 was performed in the same manner as Example 1 except that a composition for promoting hair growth was prepared by mixing 4 wt% of N-oleylethanolamine, 6 wt% of ethanol, 85 wt% of polypropylene glycol (Mw=76.09), and 5 wt% of water.

**[Table 1]**

| Effect of hair growth | | | | | |
|---|---|---|---|---|---|
| | Composition ratio of composition for promoting hair growth (wt%) | | | | Evaluation of hair growth effecta |
| | NOE | Glycol | Ethanol | Water | |
| Example 1 | 5 | 50 | 30 | 15 | ***** |
| Comparative Example 1 | 0 | 0 | 100 | 0 | * |
| Comparative Example 2 | 5 | 0 | 95 | 0 | ** |
| Comparative Example 3 | 4 | 18 | 43 | 35 | *** |
| Comparative Example 4 | 4 | 85 | 6 | 5 | *** |

| | | | | | |
|---|---|---|---|---|---|
| ^{a} criteria for evaluation of hair growth effect *****: Very Good, ****: Good, ***: Average, **: Bad, *: Very Bad | | | | | |

It could be confirmed from the results that when the composition for promoting hair growth including NOE and glycol according to a composition ratio of the present invention as active components, was used, an effect of hair growth was significantly excellent. In addition, when the composition was prepared with the composition ratio out of the range of the present invention, or when the NOE and glycol as the active components were not included, the effect of hair growth was remarkably deteriorated.

*FIG. 1 illustrates a portion of results obtained by observing the mouse right before the test and the mouse when the composition was applied for 8 weeks, and specifically, FIG. 1(a) illustrates Comparative Example 1 and FIG. 1(b) illustrates Example 1. It was confirmed from the observation result that the composition for promoting hair growth according to the present invention had a significantly high activity for hair growth.

### [Example 2] Evaluation of inflammation relieving effect

A six-week old female Balb/C mouse was purchased, and had an adaptation period of about 1 week under breeding conditions of 23 °C, relative humidity of 55 %, and illumination time for 12 hours. Then, when the mouse was 7 weeks, 20µl of 12-O-tetradecanoyl phorbol 13-acetate (TPA) which is a carcinogenic promoter at a concentration of 50 µg/mL was applied to a right ear of the mouse.

After 10 minutes, the composition for promoting hair growth of Example 1 was applied to the experimental group in an amount of 20 µL. After 6 hours, the ear was cut by 6 mm punch, and a weight thereof was measured.

### [Comparative Example 5]

Comparative Example 5 was performed is the same manner as Example 2 except that no material was applied to a mouse after 1 week of adaptation period, which was a negative control group.

### [Comparative Example 6]

Comparative Example 6 was performed in the same manner as Example 2 except that no composition for promoting hair growth was applied after applying TPA.

### [Comparative Example 7]

Comparative Example 7 was performed in the same manner as Example 2 except for applying the same amount of ethanol in an amount of 20 µL to the experimental group instead of applying the composition for promoting hair growth of Example 2.

**[Table 2]**

| Result of inflammation relieving effect | | | | | | | |
|---|---|---|---|---|---|---|---|
| | TPA (µL) | Composition ratio of composition for promoting hair growth (wt%) | | | | Evaluation of inflammation relieving effect^{b} | Ear weight of mouse (mg) |
| | | NOE | Glycol | Ethanol | Water | | |
| Example 2 | 20 | 5 | 50 | 30 | 15 | ***** | 95 |
| Comparative Example 5 (Negative Control Group) | 0 | 0 | 0 | 0 | 0 | *** | 55 |
| Comparative Example 6 | 20 | 0 | 0 | 0 | 0 | * | 125 |
| Comparative Example 7 | 20 | 0 | 0 | 100 | 0 | ** | 120 |

| | | | | | | | |
|---|---|---|---|---|---|---|---|
| ^{b} criteria for evaluation of inflammation relieving effect *****: Very Good, ****: Good, ***: Average, **: Bad, *: Very Bad | | | | | | | |

As appreciated from the results of Example, it was newly found that the composition including the ceramidase inhibitor and the glycol according to the present invention as the active components, has a significantly excellent effect for hair growth that has not been known in the art, and confirmed that the composition has an excellent effect of relieving inflammation, such that the present invention was completed.

## Claims

1. A composition for promoting hair growth, preventing hair loss, or relieving inflammation, comprising:
a ceramidase inhibitor;
500 to 2000 parts by weight of glycol based on 100 parts by weight of the ceramidase inhibitor;
C1-C5 lower alcohol; and
water.

2. The composition of claim 1, wherein the ceramidase inhibitor includes N-oleylethanolamine, derivatives thereof, salts thereof, or mixtures thereof.

3. The composition of claim 1 or 2, wherein the composition contains 0.1 to 10 wt% of the ceramidase inhibitor relative to a total composition.

4. The composition of claim 1, wherein the glycol is polypropylene glycol.

5. The composition of claim 1, wherein the composition is a pharmaceutical composition or a cosmetic composition.

6. The composition of claim 1, wherein a formulation of the composition is a skin external preparation including solution, cream, ointment, paste, aerosol agent, gel or wax, or a hair external preparation including shampoo, rinse, ampoule or treatment.
